(19) Europäisches Patentamt
European Patent Office
Office européen des brevets

(11) Publication number: **0 512 527 A1**

# (12) EUROPEAN PATENT APPLICATION

(21) Application number: **92107683.2**

(22) Date of filing: **07.05.92**

(51) Int. Cl.5: **C08B 15/06**, B01D 71/12, B01D 67/00

(30) Priority: **09.05.91 JP 132168/91**

(43) Date of publication of application: **11.11.92 Bulletin 92/46**

(84) Designated Contracting States: **DE FR GB**

(71) Applicant: **TEIJIN LIMITED**
**6-7, Minamihonmachi 1-chome Chuo-ku**
**Osaka-shi Osaka 541(JP)**

(72) Inventor: **Emi, Shingo**
**22-407, Kawanakashinmachi**
**Daito-shi, Osaka(JP)**
Inventor: **Mizutani, Shoji**
**3-1-40, Yamate-cho**
**Iwakuni-shi, Yamaguchi(JP)**
Inventor: **Suzuki, Kyoji**
**232-16, Matsutomi Kamigumi**
**Shizuoka-shi, Shizuoka(JP)**
Inventor: **Ikada, Yoshito**
**2-182, Hirookadani Gokasho**
**Uji-shi, Kyoto(JP)**

(74) Representative: **Hoeger, Stellrecht & Partner**
**Uhlandstrasse 14 c**
**W-7000 Stuttgart 1(DE)**

## (54) Modified cellulose polymer, blood processor and process for producing same.

(57) A polymer produced by reacting at least part of hydroxyl groups of a cellulose polymer with a polyfunctional isocyanate group to introduce a urethane bond and directly introducing an amino group to the end of the isocyanate group or introducing an amino group to the end of the isocyanate group through a urea bond. The use of the polymer enables a blood processor to be realized, the blood processor having an improved compatibility with an organism and able to easily remove a phosphate ion.

EP 0 512 527 A1

## BACKGROUND OF THE INVENTION

### 1. Field of the Invention

The present invention relates to a modified cellulose polymer, a blood processor using that polymer and a process for producing same. More particularly, it relates to a blood processor having a higher compatibility with an organism.

### 2. Description of the Related Art

A dialysis membrane for the purification of the blood comprising regenerated cellulose has been known for many years, and although it has various problems, it is currently widely used as an artificial kidney. Accordingly, many attempts to modify the regenerated cellulose have been made.

For example, Japanese Unexamined Patent Publication (Kokai) No. 61-8105 discloses a dialysis membrane characterized by comprising a regenerated cellulose, and chemically bonded thereto, a product of a reaction of a hydroxy compound with a polyfunctional isocyanate. This publication discloses a membrane having an improved compatibility with an organism, particularly with regard to the anticoagulation of blood and the prevention of a reduction in leucocytes and anticomplementary activity. Further, Japanese Unexamined Patent Publication (Kokai) No. 61-113459 discloses a dialysis membrane of modified cellulose. This modified cellulose has a chemical structure having various substituents on the cellulose terminal hydroxyl groups linked through a hydrocarbon group, and from this view point, is chemically different from the modified cellulose used in the present invention.

In chronic renal failure patients, the removal of phosphorus is insufficient in the dialysis, but it is considered that this phosphorus cannot be removed by a usual dialysis because the phosphate ion has a charge or exhibits a behavior similar to that of a high-molecular weight solute complexed with a protein, and is a cause of acidosis.

Accordingly, an alumina gel has been administered by preference, for rectifying the acidosis, but it has been found that $A1^{3^+}$ absorbed in a very small amount is associated with encephalopathia. Therefore, the frequent use of alumina gel has become a problem, and thus the development of a membrane by which the phosphate ion can be easily removed by dialysis is desired in the art.

## SUMMARY OF THE INVENTION

Accordingly, an object of the present invention is to provide a blood processor by which a phosphate ion is easily removed. Another object of the present invention is to provide a blood processor having an excellent compatibility with an organism.

The present inventors have made extensive and intensive studies with a view to solving the above-described problems, and as a result, have found that, when use is made of a cellulose polymer, and a hydroxyl group of the cellulose polymer is reacted with a polyfunctional isocyanate group to form a urethane bond, an amino group is directly introduced into the remaining isocyanate group or an amino group is introduced into the end of the amino group through a urea bond or the like, and this has led to the completion of the present invention.

The present invention provides a modified cellulose polymer having hydroxyl groups, at least part of which is modified as represented by the following formula:

cellulose polymer skeleton-OCONH-$R^1$-$NHR^2$ [I]

wherein $R^1$ is selected from the group consisting of an alkylene group having 1 to 10 carbon atoms, a cycloalkylene group and an arylene group, $R^2$ is a hydrogen atom or -$CONHR^3$, wherein $R^3$ is -$R^4NR^5R^6$, wherein $R^4$ is selected from the group consisting of an alkylene group having 1 to 10 carbon atoms, a cycloalkylene group and an arylene group and $R^5$ and $R^6$ may be the same or different and each is a hydrogen atom or an alkyl group having 1 to 10 carbon atoms, and a polyallylamine residue.

The present invention also provides a blood processor wherein use is made of a cellulose polymer, characterized in that the cellulose polymer constituting at least a surface capable of coming into contact with a blood is a modified cellulose having hydroxyl groups, at least part of which is modified as represented by said formula [I].

The present invention also provides a process for producing a blood processor, characterized by comprising forming a hollow fiber membrane through the use of a cellulose polymer, assembling a hollow

fiber blood processor through the use of said hollow fiber membrane, bringing a solution of a polyfunctional isocyanate dissolved in a solvent incapable of dissolving said cellulose polymer into contact with said hollow fiber membrane on at least a surface capable of coming into contact with a blood to react said solution with at least part of the hydroxyl groups of said cellulose polymer and bringing a solution of a polyfunctional amine or an aqueous acidic solution into contact with said surface to aminate an isocyanate end on the unreacted side of the reacted polyfunctional isocyanate, thereby causing the cellulose polymer on at least a surface capable of coming into contact with a blood to be modified as represented by said formula [I].

Further, the present invention provides a process for producing a blood processor, characterized by assembling a hollow fiber blood processor through the use of a hollow fiber membrane produced by forming a hollow fiber membrane through the use of a cellulose polymer, bringing a solution of a polyfunctional isocyanate dissolved in a solvent incapable of dissolving said cellulose polymer into contact with said hollow fiber membrane on at least a surface capable of coming into contact with a blood to react said solution with at least part of the hydroxyl groups of said cellulose polymer and bringing a solution of a polyfunctional amine or an aqueous acidic solution into contact with said surface to aminate an isocyanate end on the unreacted side of the reacted polyfunctional isocyanate, thereby causing the cellulose polymer on at least a surface capable of coming into contact with a blood to be modified as represented by said formula [I].

## DESCRIPTION OF THE PREFERRED EMBODIMENTS

The present invention will now be described in more detail.

The term "cellulose polymer" used in the present invention may be any cellulose polymer having a hydroxyl group modified as represented by the formula [I]. Specific examples thereof include celluloses such as regenerated cellulose and cellulose produced by a cuprammonium process, cellulose esters having hydroxyl groups into at least part of which an ester, such as an ester of acetic acid, is introduced, such as cellulose diacetate, and cellulose ethers having hydroxyl groups into at least part of which an ether bond is introduced, such as methyl cellulose. Among the above, cellulose and cellulose ester are preferred, and cellulose is particularly preferred.

In the formula [I], $R^1$ represents an alkylene group having 1 to 10 carbon atoms, a cycloalkylene group or an arylene group, and $R^1$ depends on the kind of the polyfunctional isocyanate used, and may be any group derived from a polyfunctional isocyanate.

Specific examples of the polyfunctional isocyanate used in $R^1$ include 2,4-tolylene diisocyanate (TDI), 65/35 tolylene diisocyanate, 80/20 tolylene diisocyanate, 4,4'-diphenylmethane diisocyanate, dianisidine diisocyanate, tolidine diisocyanate, hexamethylene diisocyanate (HMDI), m-xylene diisocyanate, phenyl isocyanate, p-chlorophenyl isocyanate, o-chlorophenyl isocyanate, m-chlorophenyl isocyanate, 3,4-dichlorophenyl isocyanate, 2,5-dichlorophenyl isocyanate, methyl isocyanate, ethyl isocyanate, n-butyl isocyanate, n-propyl isocyanate, octadecyl isocyanate, 1,5-naphthalene diisocyanate, polymethylenepolyphenyl isocyanate, triphenylmethane triisocyanate, transvinylene diisocyanate, tris(4-phenylisocyanate thiophosphate), N,N'(4,4'-dimethyl-3,3'-diphenyl diisocyanate)uredione, 4,4',4''-trimethyl-3,3',3''-triisocyanate, 2,4,6-triphenyl cyanurate, 2,6-diisocyanate methyl caproate and isophorone diisocyanate. Other examples of the polyfunctional isocyanate include the following compounds:
HMDI Adducts such as

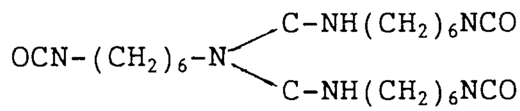 ,

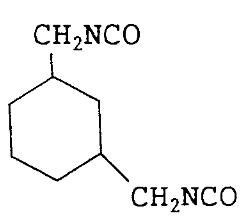 , 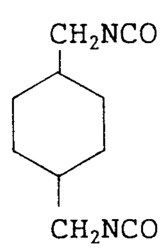 ,

OCN-[D]-NCO wherein D = $C_{36}$,

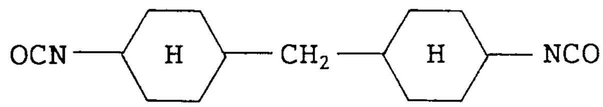 ,

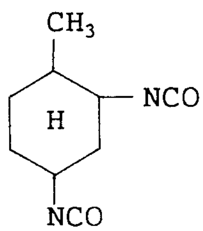 , 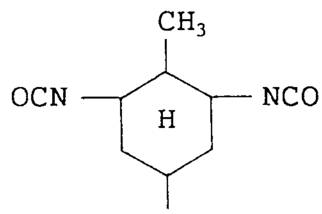 ,

polymethylene polyphenylene isocyanate (P.A.P.I.) manufactured by Up John, and polynack polyisocyanate (PPI) manufactured by Mobay.

Preferred examples of the polyfunctional isocyanate include hexamethylene diisocyanate, toluylene diisocyanate, diphenylmethane diisocyanate, dimethyldiphenyl diisocyanate, 1,5-naphthalene diisocyanate, p-phenylene diisocyanate, dicyclohexylmethane diisocyanate, isophorone diisocyanate, cyclohexyl diisocyanate, triphenylmethane triisocyanate, 2-chloro-1,4-phenyl diisocyanate, dimethoxydiphenyl diisocyanate and dimethyl-diphenylmethane diisocyanate.

In the formula [I], $R^2$ is a hydrogen atom or $-CONHR^3$, and $R^3$ is intended to mean $-R^4NR^5R^6$ or a polyallylamine residue. As with $R^1$, $R^4$ is selected from the group consisting of an alkylene group having 1 to 10 carbon atoms, a cycloalkylene group and an arylene group.

Further, $R^5$ and $R^6$ may be the same or different and each is a hydrogen atom or an alkyl group having 1 to 10 carbon atoms. Specific examples of the alkyl group having 1 to 10 carbon atoms include methyl, ethyl, n-butyl, sec-butyl, pentyl and hexyl. Preferred examples of $R^5$ and $R^6$ include a hydrogen atom and a lower alkyl group having 1 to 5 carbon atoms.

The modified cellulose polymer included in the present invention can be represented by the following formulae [I-a], [I-b] and [I-c].

cellulose polymer skeleton-$OCONH-R^1-NH_2$ [I-a]

cellulose polymer skeleton-$OCONH-R^1-NHCONH-R^4NR^5R^6$ [I-b]

cellulose polymer skeleton-$OCONH-R^1$-NHCONH-PAA [I-c]

in which PAA is a polyallylamino residue.

The modification of at least part of the hydroxyl groups in the cellulose polymer as represented by the formula [I] can be conducted by bringing a solution of the isocyanate dissolved in a solvent incapable of dissolving said cellulose polymer but capable of dissolving a polyfunctional isocyanate into contact with the cellulose polymer to conduct a reaction therebetween and aminating the isocyanate end on the unreacted side in the reacted polyfunctional isocyanate.

Any solvent incapable of dissolving the cellulose polymer but capable of dissolving the polyfunctional isocyanate may be used, but preferred examples of such a solvent include aromatic hydrocarbons such as toluene, aliphatic hydrocarbons such as n-hexane and aprotic solvents having a high polarity and a high dissolving power such as dimethylsulfoxide (DMSO).

The use of DMSO having an excellent permeability among the above-described solvents makes it possible to modify the surface of the membrane and the pore surface inside the membrane.

Examples of the polyfunctional isocyanate include hexamethylene diisocyanate, diphenylmethane diisocyanate, toluylene diisocyanate, dicyclohexylmethane diisocyanate, isophorone diisocyanate, cyclohexyl diisocyanate and triphenylmethane triisocyanate.

Although $R^1$ in the formula [I] is determined by the kind of the isocyanate, preferred examples thereof include hexamethylene diisocyanate. In the reaction, it preferably a catalyst is used; for example, preferably di-n-butyl tin dilaurate is used.

Examples of the amination of the isocyanate end on the unreacted side of the reacted polyfunctional isocyanate include a method wherein a dilute aqueous acid solution and a method wherein an aqueous solution of a polyamine having a primary amine at its one end or a polyallylamine having a primary amino group on its side chain. The polyamine, i.e., polyfunctional amine may be any one having a primary amine at its one end. Preferred examples thereof include polyamines having a tertiary amine at their other end, for example, dimethylaminopropylamine, diethylaminopropylamine and dibutylaminopropylamine.

The blood processor may be produced through the use of the modified cellulose polymer, by previously forming a hollow fiber membrane through the use of a cellulose polymer, assembling a hollow fiber blood processor using the same and converting at least part of a cellulose polymer at least on its side capable of coming into contact with a blood to a modified form represented by the following formula [I]. Alternatively, the blood processor may be produced by forming a hollow fiber membrane through the use of a cellulose polymer, subjecting the hollow fiber membrane to the above-described reaction to convert at least part of a cellulose polymer at least on its side capable of coming into contact with a blood to a modified form represented by the following formula [I] and assembling the hollow fiber blood processor through the use of the modified hollow fiber membrane.

Specific examples of the blood processor include a blood dialyzer, a plasma separator, an artificial lung and a blood filter, and further, any other type may be used. Nevertheless, a hollow fiber artificial kidney is a particularly preferable blood processor in the present invention.

The modified cellulose polymer according to the present invention is a cellulose polymer having a novel side chain unattainable in the art, and can be used as a membrane for the purification of blood having an excellent capability of removing a phosphate ion. Since it has an excellent capability of adsorbing heparin, when dialysis is conducted through the use of the hollow fiber membrane, the use of heparin in an amount larger than that used in a usual case makes it possible to provide a blood processor having an excellent anticoagulation of blood, less liable to provide residual blood, and an excellent compatibility with an organism.

The present invention will now be described in more detail with reference to the following non-limitative examples.

Example 1

Regenerated cellulose hollow fibers having an inner diameter of 200 μm and a membrane thickness of 20 μm in a wet state were bundled to form a blood processor. A solution of 3% hexamethylene diisocyanate in n-hexane as a solvent was poured into the blood processor from a side thereof capable of coming into contact with blood, to conduct a reaction at 30°C for 30 min. In this case, the blood processor was in a state such that the whole water was replaced with water and ethanol was replaced with n-hexane. In this reaction, 0.02% of di-n-butyl tin dilaurate as a solvent was added to the solution, to promote the reaction.

After the completion of the reaction, the product was washed with n-hexane to remove an unreacted isocyanate in a free form. A 2% aqueous solution of polyallylamine having a primary amino group on its side chain (obtained by preparing an aqueous solution of polyallylamine hydrochloride having a molecular weight of 10,000 manufactured by Nittobo K.K. and adjusting the pH value of the solution with NaOH to 7.7) was poured to conduct a reaction at room temperature for one hour. Thereafter, the blood processor was thoroughly washed with distilled water, and thus a blood processor useable for the dialysis of blood was provided.

Example 2

The procedure of Example 1 was repeated, to prepare a blood processor, except that a 2 wt.% aqueous dimethylaminopropylamine solution was used instead of the aqueous polyallylamine solution. The blood processor thus prepared was useable for the dialysis of blood.

Example 3

The procedure of Example 1 was repeated, to prepare a blood processor, except that the treatment was conducted through the use of a (1/1000) N hydrochloric acid solution instead of the aqueous polyallylamine solution.

Example 4

A 5% hexamethylene diisocyanate solution was prepared through the use of toluene as a solvent. The solution was poured into the blood processor assembled from a regenerated cellulose hollow fiber having an inner diameter of 200 µm and a thickness of 20 µm from the side of the processor capable of coming into contact with a blood to conduct a reaction at 30° C for 30 min. In this reaction, 0.015% of di-n-butyl tin dilaurate as a solvent was added to the solution, to promote the reaction.

After the reaction, the blood process was washed with toluene and subjected to substitution with ethanol. A 3% aqueous solution of polyallylamine (molecular weight: 60,000) was poured to conduct a reaction at room temperature for one hour. Thereafter, the blood processor was thoroughly washed with distilled water, and thus a blood processor useable for the dialysis of blood was obtained.

Example 5

A 2% hexamethylene diisocyanate solution was prepared through the use of dimethylsulfoxide (DMSO) as a solvent. A bundle of a regenerated cellulose hollow fiber having an inner diameter of 200 µm and a thickness (in a wet state) of 20 µm was immersed in the solution at 30° C for 30 min.

Thereafter, the bundle was washed with water, and the same amine as that used in Example 2 (dimethylaminopropylamine) was used to conduct amination.

The bundle was further washed with water, subjected to a glycerin treatment, and then dried to prepare a blood processor useable for the dialysis of blood.

The measurement of $DA_{phosphor}$ (dialysance of phosphor), which represents the capability of removing a phosphate ion in vitro in these cases, was conducted according to the sequence shown in Table 1.

Table 1

| Step | Procedure |
|---|---|
| Model substance Preparation of solution | • Dissolve 3 g of monosodium phosphate ($NaH_2PO_4$) in 5 liters of DW.<br>• Dissolve 14.4 g of disodium phosphate ($Na_2H_2PO_4$) in 20 liters of DW. Dissolve 225 g of NaCl in the solution in a total amount of 25 liters to prepare a B side solution having a NaCl concentration of 0.9%.<br>• Conduct a model dialysis through the use of a performance evaluation apparatus wherein a solution of 0.9% NaCl in DW was used as a D side solution to conduct sampling. |
| Amount of sampling Coloring solution | • B (bath side) original soln. 0.4 ml<br>• O (out side) dialyzed soln. 0.4 ml<br>• Add 10 ml of a coloring solution prepared by mixing phosphor B test Wako A solution with Wako B solution in a mixing ratio of 1 : 1 to 0.4 ml of the sampled solution, followed by sufficient mixing by using a test tube mixer. |
| Measurement | • 20 min after the coloring, conduct measurement at 690 nm by using a spectrophotometer. |

The calculation of DA was conducted according to the following formula.

$$DA = \frac{[B] - [C]}{[B]} \times Q_B$$

wherein [B] is reading of a spectrophotometer with respect to the original solution;
[O] is reading of a spectrophotometer with respect to the dialyzed solution; and
$Q_B$ is 200 ml/min (flow rate on side of blood solution).

## Claims

1. A modified cellulose polymer having hydroxyl groups, at least part of which is modified as represented by the following formula [I]:

   cellulose polymer skeleton-OCONH-$R^1$-$NHR^2$ [I]

   wherein $R^1$ is selected from the group consisting of an alkylene group having 1 to 10 carbon atoms, a cycloalkylene group and an arylene group, $R^2$ is a hydrogen atom or -$CONHR^3$, wherein $R^3$ is -$R^4NR^5R^6$, wherein $R^4$ is selected from the group consisting of an alkylene group having 1 to 10 carbon atoms, a cycloalkylene group and an arylene group and $R^5$ and $R^6$ may be the same or different and each is a hydrogen atom or an alkyl group having 1 to 10 carbon atoms, and a polyallylamine residue.

2. A polymer as set forth in claim 1, wherein $R^1$ is derived from a polyfunctional isocyanate selected from the group consisting of hexamethylene diisocyanate, toluylene diisocyanate, diphenylmethane diisocyanate, dimethyldiphenyl diisocyanate, 1,5-naphthalene diisocyanate, p-phenylene diisocyanate, dicyclohexylmethane diisocyanate, isophorone diisocyanate, cyclohexyl diisocyanate, triphenylmethane triisocyanate, 2-chloro-1,4-phenyl diisocyanate, dimethoxydiphenyl diisocyanate and dimethyl-diphenylmethane diisocyanate.

3. A polymer as set forth in claim 1, which is represented by the formula,

   cellulose polymer skeleton-OCONH-$R^1$-$NH_2$

   wherein $R^1$ is as defined in claim 1.

4. A polymer as set forth in claim 1, which is represented by the formula,

   cellulose polymer skeleton-OCONH-$R^1$-NHCONH-$R^4NR^5R^6$

   wherein $R^1$, $R^4$, $R^5$ and $R^6$ are as defined in claim 1.

5. A polymer as set forth in claim 1, which is represented by the formula,

   cellulose polymer skeleton-OCONH-$R^1$-NHCONH-PAA

   wherein $R^1$ is as defined in claim 1, and PAA is a polyallylamino residue.

6. A polymer as set forth in claim 1, wherein -$NHR^2$ is derived from a polyamine selected from the group consisting of dimethylaminopropylamine, diethylaminopropylamine and dibutylaminopropylamine.

7. A blood processor assembled by using a cellulose polymer, wherein the cellulose polymer constituting at least a surface capable of coming into contact with a blood is a modified cellulose having hydroxyl groups at least part of which is modified as represented by the formula [I] as defined in claim 1.

8. A blood processor as set forth in claim 7, which is one of a blood dialyzer, a plasma separator, an artificial lung, and a blood filter.

9. A blood processor as set forth in claim 8, which is a hollow fiber artificial kidney.

10. A blood processor as set forth in claim 7, wherein the cellulose polymer is in the form of a hollow fiber membrane.

11. A process for producing a blood processor, comprising forming a hollow fiber membrane through the use of a cellulose polymer, assembling a hollow fiber blood processor through the use of said hollow fiber membrane, bringing a solution of a polyfunctional isocyanate dissolved in a solvent incapable of dissolving said cellulose polymer into contact with said hollow fiber membrane on at least a surface

capable of coming into contact with a blood to react said solution with at least part of the hydroxyl groups of said cellulose polymer and bringing a solution of a polyfunctional amine or an aqueous acidic solution into contact with said surface to aminate an isocyanate end on the unreacted side of the reacted polyfunctional isocyanate, thereby causing the cellulose polymer on at least a surface capable of coming into contact with a blood to be modified as represented by the formula [I] as defined in claim 1.

**12.** A process for producing a blood processor, comprising assembling a hollow fiber blood processor through the use of a hollow fiber membrane produced by forming a hollow fiber membrane through the use of a cellulose polymer, bringing a solution of a polyfunctional isocyanate dissolved in a solvent incapable of dissolving said cellulose polymer into contact with said hollow fiber membrane on at least a surface capable of coming into contact with a blood to react said solution with at least part of the hydroxyl groups of said cellulose polymer and bringing a solution of a polyfunctional amine or an aqueous acidic solution into contact with said surface to aminate an isocyanate end on the unreacted side of the reacted polyfunctional isocyanate, thereby causing the cellulose polymer on at least a surface capable of coming into contact with a blood to be modified as represented by the formula [I] as defined in claim 1.

European Patent Office

# EUROPEAN SEARCH REPORT

Application Number

EP 92 10 7683

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | DD-A-278 495 (AKADEMIE DER WISSENSCHAFTEN DER DDR)<br>* the whole document * | 1-3,7-12 | C08B15/06<br>B01D71/12<br>B01D67/00 |
| A<br><br><br>D | EP-A-0 155 534 (AKZO GMBH)<br>* page 5, line 8 - page 6, line 25 *<br>* page 7, line 19 - line 37; claims *<br>& JP-A-61 008 105 (AKZO) | 1,2,7-12 | |
| A | US-A-3 140 256 (E. C. MARTIN ET AL.)<br><br>* column 12, line 1 - column 14, line 5 * | 1-3,7,<br>10-12 | |
| A | A.S.A.I.O. TRANSACTIONS (AMERICAN SOCIETY FOR ARTIFICIAL INTERNAL ORGANS)<br>vol. 32, no. 1, 1986, HAGERSTOWN, MD, USA<br>pages 76 - 80;<br>T. AKIZAWA ET AL.: 'Development of regenerated cellulose non-complement activating membrane for hemodialysis' | | TECHNICAL FIELDS SEARCHED (Int. Cl.5 )<br><br>C08B<br>B01D |

The present search report has been drawn up for all claims

| Place of search | Date of completion of the search | Examiner |
|---|---|---|
| THE HAGUE | 09 JULY 1992 | MAZET J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)